# EUROPÄISCHE PATENTSCHRIFT

(11) **EP 0 493 861 B1**
(45) Veröffentlichungstag und Bekanntmachung des Hinweises auf die Patenterteilung: **19.02.1997**
(21) Anmeldenummer: 91250304.2
(22) Anmeldetag: 07.11.1991
(51) Int. Cl.: A61K 31/70

(54) **Nicotinamid-adenin-dinukleodid enthaltendes Antidepressionsmittel**
Antidepressive composition containing nicotinamid-adenin-dinucleotide
Composition anti-dépressive à base de nicotinamide-adénine-dinucléotide

(30) Priorität: 04.01.1991 DE 4100361
(43) Veröffentlichungstag der Anmeldung: 08.07.1992
(73) Patentinhaber: Birkmayer, Jörg, Univ.-Prof. DDr., A-1090 Wien (AT)
(72) Erfinder: Birkmayer, Jörg, Univ.-Prof. DDr., A-1090 Wien (AT); Birkmayer, Walter, Prof. Dr., A-1090 Wien (AT)
(74) Vertreter: von Hellfeld, Axel, Dr. Dipl.-Phys.

(56) Entgegenhaltungen:
- EP-A- 0 331 620
- US-A- 3 341 412
- NEW TRENDS IN CLINICAL NEUROPHARMACOLOGY, Band 5, Nr. 1, 15. Juni 1991, Seiten 19-25; W. BIRKMAYER et al.: "The coenzyme nicotinamide adenine dinucleotide (NADH) as biological antidepressive agent"

## Beschreibung

Die Erfindung betrifft die neuartige Verwendung von Nicotinamid-adenin-dinukleotid, Nicotinamid-adenin-dinukleotidphosphat oder eines physiologisch verträglichen Salzes derselben.

Depression ist eine neuro-psychatrische Erkrankung, die das gesamte Verhalten, die Aktivität und die emotionale Befindlichkeit des Betroffenen beeinflußt. Aus einer Anzahl von Studien ist bekannt, daß Neurotransmitter, wie etwa Epinephrin (Adrenalin) und Norepinephrin (Noradrenalin), Dopamin, Serotonin oder GABA (γ-Aminobuttersäure) eine Rolle bei der Entstehung depressiver Symptome spielen (P. Riederer, W. Birkmayer und E. Neumeyer, The Tyrosin-Tryptophan-Diagram in a Longtime Study with Depressed Patients, Journal of Neural Transmission 34, 31-48 (1973) (mit Angabe weiterer Literaturstellen)). Sowohl biochemische Analysen der Gehirne von verstorbenen depressiven Patienten als auch Blut- und Urintests auf Vorläufer und Metaboliten der genannten Neurotransmitter bei Patienten, die an Depression litten, zeigten, daß offensichtlich bei diesen Patienten das Gleichgewicht der Neurotransmitter gestört ist.

Die heutzutage gängige, medikamentöse Behandlung depressiver Erkrankungen beruht im wesentlichen auf der Hemmung des Abbaus der Neurotransmitter im Neuron, um dadurch eine Anreicherung der gespeicherten Neurotransmitter zu bewirken. Dies erfolgt durchgehend durch die Verabreichung sogenannter MAO-Hemmer, die das Enzym Monoaminooxidase (MAO) blockieren, das wesentlich für den Abbau von Catecholaminen und anderen Substanzen im Neuron verantwortlich ist. Diese Behandlungsmethode hat aber trotz ihrer unbetrittenen Wirksamkeit mehrere schwerwiegende Nachteile: Zum einen beeinflußt die Hemmung des Abbaus von im Neuron vorhandenen Neurotransmittern lediglich deren Gesamtkonzentration, weniger aber das Gleichgewicht der einzelnen Neurotransmitter untereinander, so daß bspw. der Mangel an einem bestimmten Neurotransmitter aufgrund von Funktionsstörungen in der Produktion desselben nicht ausgeglichen werden kann. Die Durchführung einer Therapie mit MAO-Hemmern muß daher sehr variabel durchgeführt und auf jeden einzelnen Patienten durch eventuelle Kombination mit anderen Medikamenten speziell eingestellt werden. Eine Verabreichung dieser Medikamente kann daher nur unter ständiger ärztlicher Kontrolle erfolgen, da es schnell zu Überdosierungen kommt, die dann zu unangenehmen Nebenwirkungen, wie innere Unruhe und Schlaflosigkeit, führen können. Zum anderen handelt es sich bei der Therapie mit MAO-Hemmern um einen durchaus massiven Eingriff in die neuronalen Funktionen des Patienten, deren Auswirkungen noch nicht in vollem Umfange erforscht sind oder überschaubar sind.

Es bestand daher ein fortdauerndes Bedürfnis nach einem problemlos dosierbaren Anti-Depressionsmittel, das keine ständige ärztliche Kontrolle erfordert und keine Nebenwirkungen zeigt.

Aus der US-A-3341412 ist bekannt, daß das Diphosphopyridinnukleotid, das in dieser Literaturstelle auch als DPN bezeichnet wird, zur Bekämpfung der Symptome der Schizophrenie eingesetzt werden kann. In jeden Fall sollte die Minimaldosis 1 g DPN pro Tag ausmachen. Bei DPN handelt es sich um die oxidierte Form des Nicotinamid-adenin-dinukleotids.

Überraschenderweise hat es sich gezeigt, daß die Verwendung von Nicotinamid-adenin-dinukleotid (NADH), Nicotinamid-adenindinukleotidphosphat (NADPH) oder eines physiologisch verträglichen Salzes derselben zur Behandlung von Depressionen hervorragend geeignet ist.

Gegenstand der Erfindung ist die Verwendung von NADH, NADPH oder eines physiologisch verträglichen Salzes derselben zur Herstellung eines Arzneimittels zur Behandlung von Depressionen.

Es ist bekannt, daß Nicotinamid-adenin-dinukleotid (NADH) erfolgreich bei der Behandlung des Parkinson-Syndroms eingesetzt werden kann (EP-A-0 331 620). Die Parkinson'sche Krankheit beruht auf einer gestörten dopaminergen Neurotransmission in den Basalganglien, im allgemeinen infolge eines zunehmenden Untergangs der dopaminergen Neuronen zunächst in der Substantia nigra, im Verlauf der Krankheit auch in anderen Arealen.

Völlig überraschend hat sich nunmehr in klinischen Untersuchungen gezeigt, daß die für die Behandlung der Parkinson'schen Krankheit vorgeschlagenen Medikamente auch äußerst erfolgreich bei der Behandlung von Depressionen eingesetzt werden können, bei denen die Ursachen vielfältigster Natur sind.

Bevorzugt wird dabei das Nicotinamid-adenin-dinukleotid, das Nicotinamid-adenin-dinukleotidphosphat oder das physiologisch verträgliche Salz derselben dem Körper in einer Menge von 1 mg bis 50 mg, besonders bevorzugt 5 mg bis 12,5 mg, als Einzeldosis zugeführt.

Weitere Vorteile und Merkmale der Erfindung ergeben sich aus der nachfolgenden Beschreibung der klinischen Testreihen, die bisher zu diesem Thema durchgeführt wurden.

Eine erste Testreihe umfaßte zunächst 15 Patienten, die aus unterschiedlichsten Ursachen an Depressionen litten. Zur Beurteilung der Erkrankung wurde die sogenannte Birkmayer-Skala für Depressionen verwendet, die sich aus Tabelle 1 unten ergibt. Die Patienten wurden vor der Therapie und nach einem bestimmten Behandlungszeitraum untersucht.

In einer ersten Gruppe umfaßten die 15 Patienten 5 Frauen und 10 Männer. Der jüngste Patient war 26, der älteste 76. Alle Patienten erhielten die reduzierte Form von Nicotinamid-adenin-dinukleotid (NADH) entweder parental oder oral. Die Ergebnisse sind in Tabelle 2 zusammengestellt. In einer zweiten Gruppe von weiteren 15 Patienten, von denen 8 Frauen und 7 Männer waren (jüngster Patient 25, ältester Patient 68) wurde die reduzierte Form von Nicotinamid-adenin-dinukleotidphosphat (NADPH) verabreicht, auch entweder parental oder oral. Die Ergebnisse sind in Tabelle 3 unten dargestellt.

Die Zeiträume für die Therapie betrugen von 2 Wochen bis zu 12 Wochen. Die Einzelbewertung aufgrund der Birkmayer-Skala vor der NADH- bzw. NADPH-Behandlung betrug 19 bis 41 der möglichen 80 Punkte. Die Untersuchung nach der Therapie zeigt eine Verbesserung von 63 bis 97 % der ursprünglich vor der Therapie festgestellten Werte. Drei (1. Gruppe) bzw. fünf (2. Gruppe) der Patienten kehrten zu einem fast vollständig normalen Verhalten zurück, mit lediglich einem Punkt in der 80 Punkte-Skala, wobei 0 Punkte ein absolut normales Verhalten kennzeichnen.

Insgesamt zeigen die Ergebnisse, daß die Behandlung von Depressionen mit NADH oder NADPH bereits nach sehr kurzer Zeit überzeugende Verbesserungen des Krankheitsbildes zeigen.

Jeweils 6 Patienten in jeder Untersuchungsgruppe erhielten das NADH bzw. NADPH intravenös, 2 intramuskulär und der Rest oral. Die Dosierung schwankte zwischen 1 und 50 mg, wobei die besten Ergebnisse bei Verabreichung von 5 bis 12,5 mg erreicht wurden. Die intravenöse Verabreichung erfolgte in Form einer Infusion von 10 mg NADH bzw. NADPH in 200 ml Elehest. Die intravenöse Verabreichung erfolgte 3mal wöchentlich. Die orale Verabreichung wurde täglich oder jeden zweiten Tag vorgenommen. Aufgrund der vorliegenden Ergebnisse scheint die Art der Verabreichung keinen wesentlichen Einfluß auf die Verbesserung des Krankheitsbildes zu haben. Nebeneffekte sind nicht beobachtet worden (auch nicht nach 12wöchiger Behandlung).

Der wesentliche Vorteil von Nicotinamid-adenin-dinukleotid und Nicotinamid-adenin-dinukleotidphosphat gegenüber den gegenwärtig bevorzugten Anti-Depressionsmitteln, d.h. den MAO-Hemmern, besteht im wesentlichen in seiner biologischen Herkunft. Nicotinamid-adenin-dinukleotid bzw. Nicotinamid-adenin-dinukleotidphosphat tritt im menschlichen Körper natürlich auf und ist Enzym-Kofaktor für eine ganze Anzahl von Dihydrogenasen. Die Verabreichung von Nicotinamid-adenin-dinukleotid, Nicotinamid-adenin-dinukleotidphosphat oder eines physiologisch verträglichen Salzes derselben greift daher nicht direkt in die neuralen Funktionen des Menschen ein, sondern begünstigt offenbar auf bisher noch nicht geklärte Weise die Wiederherstellung eines gestörten Gleichgewichts der Neurotransmitter. Eine Behandlung mit Nicotinamid-adenin-dinukleotid, Nicotinamid-adenin-dinukleotidphosphat oder einem physiologisch verträglichen Salz derselben ist daher problemlos zu steuern und kann auch ohne ärztliche Kontrolle nicht zu Überdosierungen und damit verbundenen unangenehmen Nebenwirkungen führen, wie sie von den MAO-Hemmern bekannt sind.

Die in der vorstehenden Beschreibung sowie in den Ansprüchen offenbarten Merkmale der Erfindung können sowohl einzeln als auch in beliebiger Kombination für die Verwirklichung der Erfindung in ihren verschiedenen Ausführungsformen wesentlich sein.

**Tabelle 1**

| Bewertungsskala für Depressionen (Prof. Dr. W. Birkmayer) | | | | | |
|---|---|---|---|---|---|
| | | Bewertungszahl | | | |
| Bewertungsparameter | | 0 | 1 | 2 | 3 |
| 1. | Lustlos | | | | |
| 2. | Freudlos | | | | |
| 3. | Interesselos | | | | |
| 4. | Antriebslos | | | | |
| 5. | Konzentrationsmangel | | | | |
| 6. | Verminderte Leistungsfähigkeit | | | | |
| 7. | Schlaflos | | | | |
| 8. | Appetitlos | | | | |
| 9. | Gewichtsabnahme | | | | |
| 10. | Obstipation | | | | |
| 11. | Libidoverlust | | | | |
| 12. | Abendliche Remission | | | | |
| 13. | Zwangsgrübeln | | | | |
| 14. | Allgemeiner Pessimismus | | | | |
| 15. | Selbstvorwürfe | | | | |
| 16. | Schuldgefühle | | | | |
| 17. | Angst | | | | |
| 18. | Suizidneigung | | | | |
| 19. | Hypochondrische Beschwerden | | | | |
| 20. | Gedanken über die Sinnlosigkeit des Lebens | | | | |

**Tabelle 2**

| NADH-Therapie | | | | | | | |
|---|---|---|---|---|---|---|---|
| Nr. | Geschlecht | Alter | Behandlung | Behandlungsdauer | Vorher | Nachher | Verbesserungen |
| 1 | M | 59 | 10 mg i.v. 3mal/Woche | 5 Wochen | 29 | 1 | 96,5 |
| 2 | M | 53 | 6 mg i.m. täglich | 2 Wochen | 32 | 10 | 68,2 |
| 3 | M | 26 | 6 mg i.v. wöchentlich | 3 Wochen | 31 | 3 | 90,3 |
| 4 | F | 51 | 5 mg oral 3mal/woche | 5 Wochen | 41 | 5 | 87,8 |
| 5 | M | 49 | 10 mg i.v. 3mal/Woche | 4 Wochen | 26 | 1 | 96.2 |
| 6 | M | 71 | 10 mg oral täglich | 4 Wochen | 24 | 18 | 66,7 |
| 7 | M | 76 | 6 mg i.v. täglich | 3 Wochen | 19 | 3 | 84,2 |
| 8 | M | 49 | 10 mg i.v. | 3 Wochen | 24 | 3 | 87,5 |
| 9 | M | 47 | 5 mg oral täglich | 12 Wochen | 31 | 11 | 64,5 |
| 10 | F | 69 | 12,5 mg i.v. 3mal/Woche | 5 Wochen | 19 | 7 | 63,2 |
| 11 | M | 60 | 5 mg i.m. 3mal/Woche | | 28 | 1 | 96,5 |
| 12 | F | 36 | 5 mg oral alle 2 Tage | 3 Wochen | 31 | 3 | 90,3 |
| 13 | F | 55 | 1 mg i.m. 3mal/Woche | 6 Wochen | 35 | 4 | 88,6 |
| 14 | F | 29 | 50 mg oral täglich | 3 Wochen | 32 | 6 | 81,25 |
| 15 | M | 42 | 20 mg i.v. 3mal/Woche | 10 Wochen | 28 | 9 | 67,9 |

**Tabelle 3**

| NADPH-Therapie | | | | | | | |
|---|---|---|---|---|---|---|---|
| Nr. | Geschlecht | Alter | Behandlung | Behandlungsdauer | Vorher | Nachher | Verbesserung |
| 1 | M | 51 | 10 mg i.v. 3mal/Woche | 2 Wochen | 28 | 1 | 96,5 |
| 2 | M | 25 | 6 mg i.m. täglich | 3 Wochen | 19 | 6 | 68,4 |
| 3 | M | 42 | 6 mg i.v. wöchentlich | 5 Wochen | 24 | 5 | 79,2 |
| 4 | F | 37 | 5 mg oral 3mal/Woche | 4 Wochen | 41 | 10 | 75,6 |
| 5 | F | 60 | 10 mg i.v. 3mal/Woche | 3 Wochen | 32 | 4 | 87,5 |
| 6 | M | 65 | 10 mg oral täglich | 2 Wochen | 28 | 3 | 89,3 |
| 7 | M | 68 | 6 mg i.v. täglich | 3 Wochen | 35 | 1 | 97,1 |
| 8 | M | 48 | 10 mg i.v. täglich | 4 Wochen | 32 | 11 | 65,6 |
| 9 | M | 61 | 1 mg oral täglich | 5 Wochen | 28 | 3 | 89,3 |
| 10 | F | 53 | 50 mg i.v. 3mal/Woche | 2 Wochen | 31 | 1 | 96,8 |
| 11 | F | 60 | 5 mg i.m. 3mal/Woche | 10 Wochen | 29 | 2 | 93,1 |
| 12 | F | 48 | 3 mg oral alle 2 Tage | 4 Wochen | 20 | 1 | 95 |
| 13 | F | 53 | 7 mg i.m. 3mal/Woche | 3 Wochen | 40 | 9 | 77,5 |
| 14 | F | 27 | 15 mg i.v. 3mal/Woche | 10 Wochen | 31 | 10 | 67,7 |
| 15 | F | 67 | 50 mg oral alle 2 Tage | 3 Wochen | 27 | 6 | 77,8 |

## Patentansprüche

1. Verwendung von NADH, NADPH oder eines physiologisch verträglichen Salzes derselben zur Herstellung eines Arzneimittels zur Behandlung von Depressionen.

2. Verwendung nach Anspruch 1, dadurch gekennzeichnet, daß das Nicotinamid-adenin-dinukleotid, das Nicotinamid-adenin-dinukleotidphosphat oder das physiologisch verträgliche Salz derselben dem Körper in einer Menge von 1 mg bis 50 mg als Einzeldosis zugeführt wird.

3. Verwendung nach Anspruch 2, dadurch gekennzeichnet, daß die Einzeldosis 5 mg bis 12,5 mg beträgt.

## Claims

1. A use of NADH, NADPH or a physiologically compatible salt thereof for the preparation of a medicament for the treatment of depressions.

2. The use according to claim 1, characterized in that the nicotinamide-adenine-dinucleotide, the nicotinamide-adenine-dinucleotide phosphate, or physiologically compatible salt thereof is supplied to the body in a quantity of 1 mg to 50 mg as a single dose.

3. The use according to claim 2, characterized in that the single dose is 5 mg to 12,5 mg.

## Revendications

1. Utilisation de NADH, NADPH ou d'un sel physiologiquemen toléré de ces derniers pour la préparation d'un médicament destiné au traitement de dépressions.

2. Utilisation selon la revendication 1, caractérisée en ce que le nicotinamide-adénine-dinucléotide, le nicotinamide-adénine-dinucléotide-phosphate ou le sel physiologiquement toléré de ces derniers est apporté à l'organisme en une quantité de 1 mg à 50 mg sous forme de dose unique.

3. Utilisation selon la revendication 2, caractérisée en ce que la dose unique est de 5 mg à 12,5 mg.
